(19) Europäisches Patentamt
European Patent Office
Office européen des brevets

(11) Publication number: 0 392 680 A2

# (12) EUROPEAN PATENT APPLICATION

(21) Application number: **90303007.0**

(22) Date of filing: **20.03.90**

(51) Int. Cl.[5]: **C07C 225/22, C07C 317/22, C07C 317/36, C07D 487/04, C08G 73/10**

(30) Priority: **13.04.89 GB 8908393**
**13.04.89 GB 8908395**

(43) Date of publication of application:
**17.10.90 Bulletin 90/42**

(84) Designated Contracting States:
**AT BE CH DE ES FR GB IT LI LU NL SE**

(71) Applicant: **IMPERIAL CHEMICAL INDUSTRIES PLC**
**Imperial Chemical House, Millbank**
**London SW1P 3JF(GB)**

(72) Inventor: **Young, Stella Margaret**
**109 Aldenham Road**
**Guisborough, Cleveland(GB)**
Inventor: **Mayo, Richard Andrew**
**26 Hitchin Road**
**Weston, Hertfordshire(GB)**

(74) Representative: **Graham, John George et al**
**Legal Dept. Patents PO Box 6 Bessemer Road**
**Welwyn Garden City Hertfordshire AL7 1HD(GB)**

## (54) Aromatic compounds.

(57) Aromatic compounds of formula I
Z Ph′ X Ph Y Ar Y Ph X Ph′ Z I
where
X is selected from -CO-, -SO- or $-SO_2-$;
Y is selected from -O- or -S-;
Z is $-NH_2$ or a maleimide radical;
Ar is a phenylene unit or a polyaromatic group containing at least two aromatic units which are either linked directly together or are linked by a class selected from -CO-, -SO-, $-SO_2-$, -O-, -S-, $-C(CH_3)_2$ and other aliphatic groups;
Ph is 1,4-phenylene; and
Ph′ is 1,3-phenylene when Z is $-NH_2$ and Ph′ is 1,3 - or 1,4 - phenylene when Z is a maleimlde radical.

Polymers comprise a repeat unit of formula
$- D - Z^3 -$
where D is a divalent aromatic radical derived from a compound of formula I, in which Z is $-NH_2$ and Ph′ is 1,3-phenylene, and $Z^3$ is

EP 0 392 680 A2

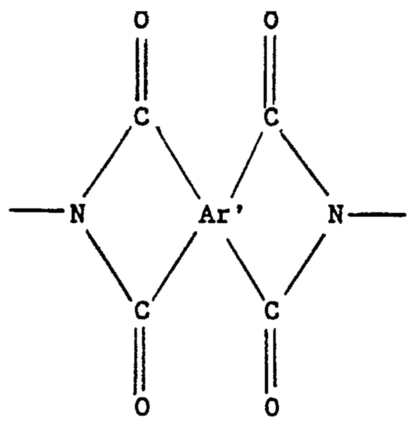

or

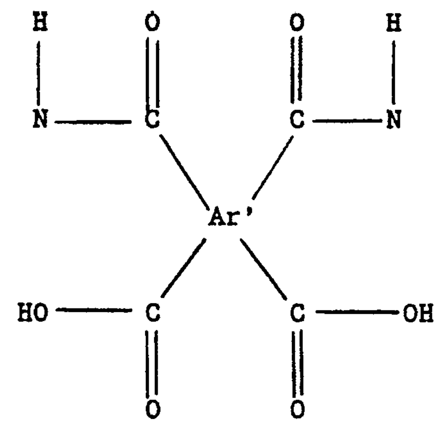

where $Ar'$ is a tetravalent aromatic radical derived from a dianhydride or a corresponding derivative thereof. Up to 50 mole % of the radicals D can be replaced by divalent organic radicals derived from diamines other than those falling within formula I.

## Aromatic Compounds

This invention relates to aromatic compounds, particularly aromatic diamines and aromatic bismaleimides, and to polymers which may be produced from the aromatic compounds. US Patent 4 746 718 describes aromatic diamines of formula

$H_2N\ Ph^1\ X^1\ Ph^1\ Y^1\ Ar^1\ Y^1\ Ph^1\ X^1\ Ph^1\ NH_2$

where $X^1$ is -CO- or $-SO_2-$, $Y^1$ is -O- or -S-, $Ar^1$ comprises from 1 to 80 interconnected aryl radicals and $Ph^1$ is 1,4 phenylene and methods for their preparation. French Patent 2 611 710 describes aromatic diamines of formula

$H_2N\ Ph^2\ SO_2\ Ph^2\ Y^2\ V\ Y^2\ Ph^2\ SO_2\ Ph^2\ NH_2$

where $Ph^2$ can be a phenylene unit, $Y^2$ is a group which contains an oxygen or sulphur atom and V can be a phenylene unit and methods for their preparation. The method of preparation disclosed only results in the formation of aromatic diamines in which $Ph^2$ is 1,4-phenylene.

According to the present invention, there is provided an aromatic compound of formula I

$Z\ Ph'\ X\ Ph\ Y\ Ar\ Y\ Ph\ X\ Ph'\ Z$ I

wherein:-

X is -CO-, -SO- or $-SO_2-$;

Y is -O- or -S-;

Z is $-NH_2$ or a maleimide radical;

Ar is a phenylene unit or a polyaromatic group containing at least two aromatic units which are either linked directly together or are linked by -CO-, -SO-, $-SO_2-$, -O-, -S-, $-C(CH_3)_2$ or other aliphatic groups;

Ph is 1,4-phenylene; and

$Ph'$ is 1,3-phenylene when Z is $-NH_2$ and $Ph'$ is 1,3 - or 1,4 - phenylene when Z is a maleimide radical.

By "directly linked together" we mean that the aromatic units are linked by a direct link between the rings as in biphenylene or terephenylene or are linked in a polynuclear aromatic system as in naphthalene. Examples of Ar groups include:-

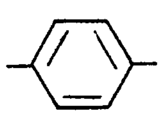
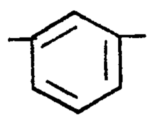
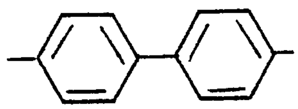
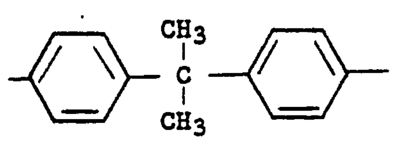

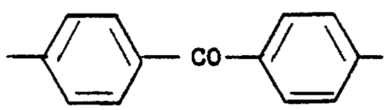
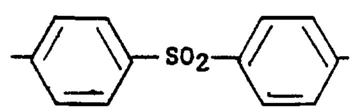
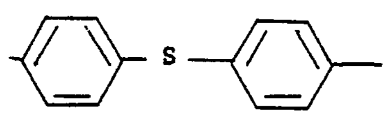

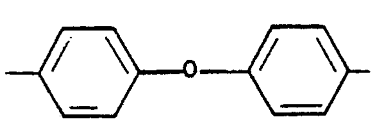
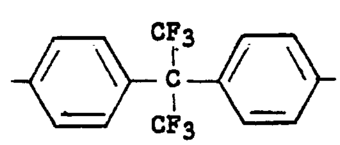
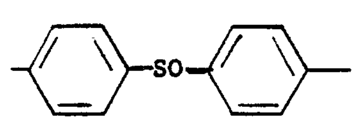

The Ar groups may be substituted, for example by $C_1$ to $C_4$ alkyl or perfluoroalkyl groups, aryl groups eg phenyl, halogen and nitro groups.

The invention includes processes for making the compounds.

Preferably, compounds of formula I in which Z is $-NH_2$ are prepared by condensation of a halobenzene of formula II with a bisphenol of formula III:-

$H_2\ N\ Ph'\ X\ Ph\ Z'$ II

HO Ar OH III

wherein $Z'$ is halogen, especially F or Cl. Such condensations are effected in the presence of a base.

Alternatively, compounds of formula I in which Z is $-NH_2$ may be prepared by the condensation of the

nitro-halide compound and the bisphenol followed by reduction of the nitro groups to amine groups; or by condensation of the amino-hydroxy compound and a dihalide provided the halogen groups are activated, eg by $-SO_2-$ or -CO- or by Cu catalyst; or by acylation of a compound of formula Ph Y Ar Y Ph with a compound of formula $O_2$ N Ph′ X Z′, Ph, Ph′, X, Y and Z′ being as hereinbefore defined, in the presence of an acid capable of catalysing the reaction, eg a Friedel-Crafts catalyst such as aluminium chloride, followed by reduction of the nitro groups to amine groups. Preferably, the reduction is carried out using palladium on charcoal and cyclohexene.

Compounds of formula I in which Z is a maleimide radical are prepared by a condensation reaction between the corresponding diamine and maleic anhydride or a reactive derivative thereof.

By "reactive derivative" we mean a derivative of maleic acid which will condense with a diamine to form a maleimide and, as is well understood in the art, such derivatives include maleic acid itself, esters and acid chlorides thereof.

Preferably, however, the bismaleimides of the invention are prepared using maleic anhydride.

The processes of the invention are preferably carried out using a dipolar aprotic solvent such as dimethylacetamide, N-methylpyrollidone, dimethylsulphoxide and dimethylformamide. Mixtures of dipolar aprotic solvents may be used. Mixtures of solvents one of which is not a dipolar aprotic solvent may be used.

In preferred compounds of formula I in which Z is a maleimide radical, Ph′ is 1,3-phenylene.

Examples of preferred compounds of formula I according to the invention are:-

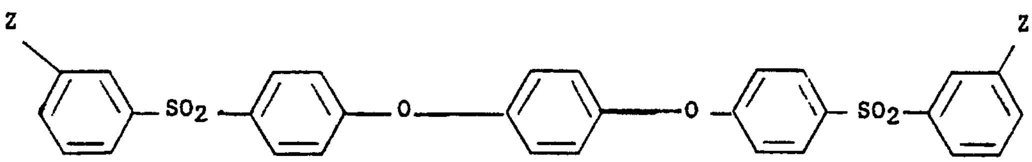

IV

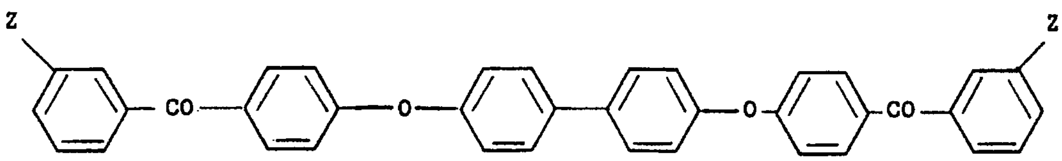

V

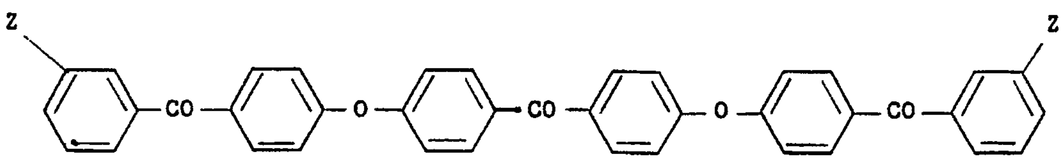

VI

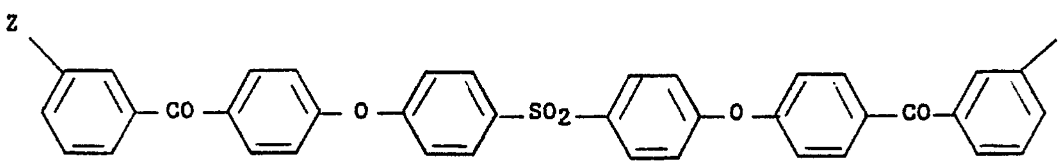

VII

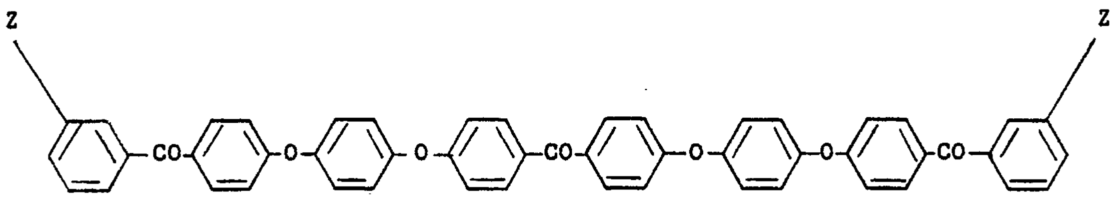

VIII

The novel diamines of the present invention are useful as crosslinking agents in epoxy resin systems and as precursors, for example for novel polyamides and polyimides and bismaleimides.

The novel bismaleimides of the present invention are useful in manufacturing reinforced articles. The bismaleimides of the invention can be formed, moulded, applied to fibrous materials, for example short or continuous fibres (whether unidirectional or woven) such as carbon, glass and organic or inorganic filaments or other additives at temperatures well below the temperature at which the bismaleimide crosslinks with itself to form a cured article. Thus, according to another aspect of the present invention, an article comprises a thermoset bismaleimide according to the first aspect of the invention.

According to a further aspect of the present invention, there is provided a thermoplastic aromatic polymer which comprises a repeat unit of formula

$- D - Z^3 -$

wherein D is a divalent aromatic radical derived from compounds of formula I,

Z Ph′ X Ph Y Ar Y Ph X Ph′ Z     I

as hereinbefore defined and in which Z is $-NH_2$, and Ph′ is 1,3 phenylene and $Z^3$ is

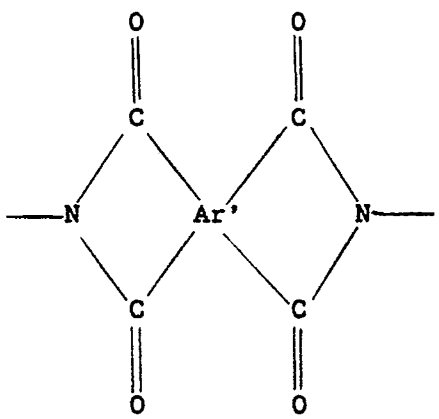

or

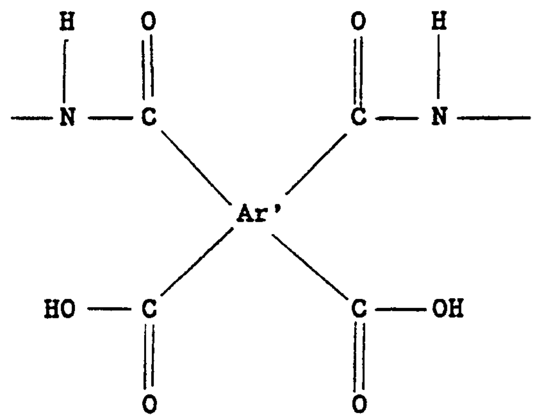

in which Ar′ is a tetravalent aromatic radical derived from a dianhydride or corresponding derivative thereof; and wherein up to 50 mole % of the radicals D are optionally replaced by divalent organic radicals derived from diamines other than those falling within formula I.

Also according to the present invention, there is provided a process of forming a thermoplastic aromatic polymer as defined in the preceding paragraph which comprises reacting a dianhydride of formula

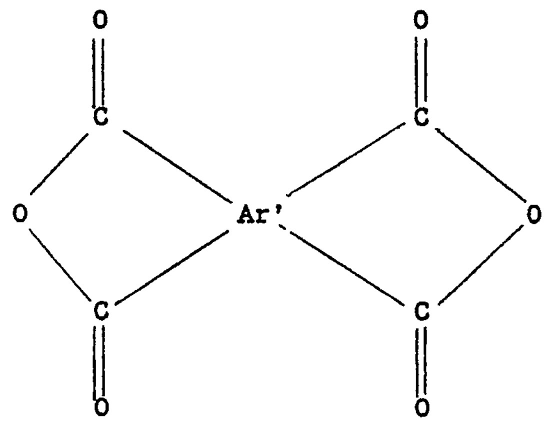

or a corresponding derivative thereof, where Ar′ is as hereinbefore defined, with at least one compound of formula I, where Z is $-NH_2$ and, optionally, with up to 50 mole % of at least one other diamine.

By "derivative" we mean a derivative of a tetracarboxylic acid which will condense with a compound of formula I, where Z is $-NH_2$, to form a polymer and, as is well understood in the art, such derivatives include the acid itself, diesters and acid chlorides.

Examples of preferred dianhydrides usable to form polymers of the invention are:-
pyromellitic dianhydride, benzophenone dianhydride, biphenyl dianhydride, diphenyl ether dianhydride, diphenyl sulphone dianhydride, bis (dicarboxyphenyl) hexafluoropropane dianhydride and 1,4,5,8- naphthalene dianhydride.

The other diamines which can be used to the extent of replacing up to 50 mol % of the compounds of formula I, where Z is $-NH_2$, may be selected from any diamine, preferably an aromatic diamine, such as 4,4′diaminodiphenylsulphone, 3,3′diaminodiphenylsulphone, 3,4′diaminodiphenylsulphone, 4,4'diaminobenzophenone, 3,3'diaminobenzophenone, 3,4′ diaminobenzophenone, 4,4′diaminophenylether, 3,3′diaminodiphenylether, 3,4′ diaminodiphenylether 4,4′diaminodiphenylmethane, m-phenylene diamine, p-phenylene diamine, o-phenylene diamine, 4,4′bis-(4 amino phenoxy)diphenylsulphone and 1,3 bis-(3 amino phenoxy)benzene.

At least 50 mol %, and preferably at least 60 mole %, of the total diamine content used to form polyimides according to the invention should be a compound of formula I where Z is $-NH_2$. Other diamines may be present, and may be usefully employed when it is required to increase the glass transition temperature (Tg) of the polyimide produced. Increases in Tg may have adverse effects on processability. On the other hand chemical and environmental resistance may be improved and the ultimate choice of co-reactant amine, if any, will depend on the intended application.

Preferably, polymers of the invention are polyimides. However, the invention also includes polymers which are polyamic acids, ie polyimide precursors, which, for example, find use as adhesives, varnishes, Impregnation materials and the like. In such applications, the polyamic acid is applied to a component as a solution, the acid then being dehydrated to convert it into the corresponding polyimide.

Thus, according to another aspect of the present invention, an article comprises a polyimide formed by the in situ dehydration of a polyamic acid according to the invention.

The preferred polymers of the invention are essentially linear even when fully ring closed and the preferred polyimides are substantially free from residual reactive groups such as anhydride, acid, amide or amine end groups. By "essentially linear" is meant that although the polymer may be branched to a limited extent it must be essentially free from cross-links which would render it no longer melt processible at temperatures below the decomposition temperature of the polyimide.

By being "substantially free from residual reactive groups such as anhydride, acid, amide or amine end groups", a melt stable polymer will be obtained. Preferably, the number of functional end groups in the polyimide is less than 1.5 per 100 repeat units in the polyimide. The preferred process for making a polyimide according to the invention which is melt processible comprises reacting a slight excess of either the dianhydride or the diamine components and end-capping with a suitable monofunctional reagent. It is preferred that an excess of terminal amine groups are present in the initial reactants and that the terminal amine groups are end-capped, using, for example, phthalic anhydride or a phthalic acid ester so that the polyimide end groups are essentially all unsubstituted aromatic groups.

The condensation reaction between the dianhydride and the diamine is preferably carried out in a dipolar, aprotic solvent such as dimethylacetamide, N-methylpyrollidone, dimethylsulphoxide and dimethyl

formamide. Mixtures of dipolar aprotic solvents may be used. Mixtures of solvents one of which is not a dipolar aprotic solvent may be used.

It is advantageous to complete the cyclisation reaction and to end cap the polymer whilst the polymer is in solution. Preferably a high boiling solvent is used to keep the polyimide in solution until the cyclisation reactions are completed at elevated temperature, in excess of 100 °C.

In order to obtain products of the required melt stability it is generally necessary to use an excess of end-capping agent over that required for theoretical conversion of the calculated molar excess of functional end groups. When the reactants contain an excess of amine reactant the concentration of monofunctional end capping agents, such as phthalic anhydride, must be in excess of the calculated amine functionalities. It may be necessary to use as much as a 200% molar excess of end-capping agent to obtain the required melt stability when the end-capping reaction is done under conditions where the cyclised polymer comes out of solution as it is formed or when cyclisation is performed in solution but at low temperature. When the polymer is kept in solution at elevated temperature during cyclisation much less end-capping agent can be used to obtain satisfactory melt stability. Under these conditions a 50% molar excess gives excellent stability and as little as a 25% molar excess can be used.

An advantage of the polymers according to the present invention is that because the chemical reactions are virtually complete little or no provision needs to be made for removing evolved condensation by-products.

The reaction conditions can be controlled to produce polyimides over a wide range of molecular weight.

Preferred polyimides according to the invention exhibit advantageously low Tg and melt viscosity. Consequently, such preferred polyimides are thermoformable in the sense of being repeatedly formable when held at elevated temperature. Thus, not only can shaped articles be formed at processing temperatures of the order of 400° or less, but the material remains sufficiently themoformable, that is the polymer remains sufficiently linear to be thermoformed in further high temperature thermoforming operations. This property is advantageous because it is possible to supply a fabricator with a composite in which the chemistry (evolution of volatiles) has been completed, rather than requiring the fabricator to deal with the problem of performing the chemistry and removing the volatiles and voids during fabrication of shaped parts.

The polyimides of the invention can be used to form films or fibres, particularly by melt extrusion of the polymer.

The polyimides of the invention may be reinforced with 2 to 70% by weight fibrous materials, for example short or continuous fibres such as carbon, glass and organic or inorganic filaments such as alumina fibres. Continuous fibres may be provided as unidirectional filaments or in woven form.

Stabilisers, fillers, pigments and other additives including short fibre reinforcing materials (ie fibres of length less than 3 mm) may also be employed.

Thus, also according to the present invention, an article comprises a polyimide according to the invention.

The invention is illustrated by reference to the following Examples.

## Example I

4-fluoro-3′-aminodiphenylsulphone (0.1 mole, 25.1g) was charged into a 250 ml conical flask fitted with an $N_2$ inlet, a PTFE coated magnetic stirrer, a thermometer and a condenser. Hydroquinone (0.05 mol, 5.5g) and N-methylpyrrolidone (50 ml) were added to the flask. The contents of the flask were stirred whilst potassium carbonate (0.1 mol, 13.8g) was added, which caused an immediate yellow coloration. The temperature of the contents was raised to and held at 100 °C for one hour and then raised to and held at 170 °C for three hours. The contents of the flask were allowed to cool then precipitated into 150 ml of rapidly stirred demineralised water. The crude product was recovered by filtration, washed twice with demineralised water and then recrystallised from aqueous dimethylacetamide.

Yellow crystals were recovered in 95% yield which had a melting point of 156-158 °C.

The product was identified by nuclear magnetic resonance (nmr) spectroscopy as being the compound of formula IV in which Z is $-NH_2$.

## Example II

The assembly described in Example I was charged with 4-fluoro-3′-aminobenzophenone (0.08 mol,

17.22g), 4,4' biphenol (0.04 mol, 7.45g), N-methyl pyrrolidone (40 ml) and potassium carbonate (0.08 mol, 11.06g). The mixture was stirred and the temperature was raised to and held at 100 °C for 1 hour and then raised and held at 170 °C for 5½ hours. The flask contents were cooled, and precipitated into 100 mls of demineralised water to give a yellow solid which was washed with demineralised water, filtered and dried to give 22.61 grams (91.7% yield) of crude product melting at 184-186 °C.

The product was identified by nmr spectroscopy as being the compound of formula V in which Z is $-NH_2$.

## Example III

(i) 4,4'-Difluorobenzophenone (109g, 0.5m) and phenol (95.8g, 1m) were dissolved in N-methyl pyrollidone (500ml) in a reaction flask. Potassium carbonate (72.45g, 0.525m) was added to the flask and mixture heated to and held at 180 °C for 3 hours. The reaction mixture was cooled and then quenched into sodium bicarbonate solution. The resulting precipitate was washed with 500ml of water three times and gave white needles (171.4g, yield 93.6%) melting at 147.2 °C. The ir spectrum and mass spectrum of the product was measured. The product was identified as 4,4'-diphenoxybenzophenone.

(ii) Aluminium chloride (47g, 0.35m) was added in 5 gram portions to a stirred suspension of 4,4'-diphenoxybenzophenone (36.3g, 0.1m) and 3-nitrobenzoyl chloride (41.8g, 0.22m) in 1,2 dichloroethane (100ml) in a reaction flask. As the aluminium chloride was added to the reaction solution a yellow solution formed initially. This changed colour during the course of addition of the aluminium chloride eventually turning brown. After all the aluminium chloride had been added the reaction mixture was refluxed for 2½ hours at a temperature of 80 °C to complete the reaction and thereby remove hydrogen chloride. The reaction mixture was cooled and then poured into acidified ice water (1 litre). Thereafter the dichloroethane was boiled off the reaction mixture and the resulting solid washed in methanol to remove aluminium salts. The product was a white solid (59.4g, yield 89%) melting at 229.7 °C. The ir spectrum of the product was measured. Mass spectroscopy gave an m/e of 664. The product was identified as bis 4,4'(3-nitrobenzoyl-4-phenoxy)benzophenone.

(iii) Bis 4,4'-(3-nitrobenzoyl-4-phenoxy)benzophenone (120g, 0.18m), cyclohexene (250ml, 2.475m), palladium/charcoal (5% Pd/C) (7.19g) and dimethylformamide (1 litre) were charged into a reaction flask and stirred. stirrer. The reaction mixture was refluxed for 3 hours until thin layer chromatography (performed on silica gel plates $60F_{245}$ plates (aluminium) in solvent systems A (petroleum ether (60-80 °C):ethyl acetate) showed the reaction was complete. Then the cyclohexene and water were distilled off and the Pd/C was removed by filtration. Water was added to the filtrate to yield a green/yellow solid which on recrystallisation gave a yellow solid (81.5g, yield 75%) melting at 221.4 °C. The ir spectrum was measured.

The product was identified as being the compound of formula VI in which Z is $-NH_2$.

## Example IV

The procedure used in Example III was repeated except for the following differences

(i) 4,4'-Difluorobenzophenone was replaced by 4,4'-difluorodiphenylsulphone and the reaction was heated and held at 180 °C for 2 hours. The reaction gave a white solid (yield 89.4%) melting at 141.9 °C and having an m/e of 402. The product was identified as 4,4'-diphenoxydiphenylsulphone.

(ii) 4,4'-Diphenoxybenzophenone was replaced by 4,4'-diphenoxy diphenylsulphone. The reaction gave an "off-white" coloured solid (yield 93%) melting at 189.2 °C and having an m/e of 700. The product was identified as bis-4,4'-(3-nitrobenzoyl-4-phenoxy)diphenylsulphone.

(iii) Bis-4,4'-(3-nitrobenzoyl-4phenoxy)benzophenone was replaced by bis-4,4'-(3-nitrobenzoyl-4-phenoxy)diphenylsulphone and the reaction refluxed for 4 hours. The reaction gave a yellow solid (yield 56%) melting in the range 159.6-163.5 °C and having an m/e of 640.

The product was identified as being the compound of formula VII in which Z is $-NH_2$.

## Example V

The procedure used in Example III was repeated except for the following differences

(i) Phenol was replaced by 4-phenoxyphenol and the reaction was heated and held at 180 °C for 5 hours. The reaction gave a white solid (yield 89.1%) melting at 198.6 °C and having an m/e of 548. The

product was identified as a compound of formula:- H Ph O Ph O Ph CO Ph O Ph O Ph H.

(ii)4,4′-Diphenoxybenzophenone was replaced by the product of step (i). The reaction gave an "off-white" coloured solid (yield 92.9%) melting at 247.5° C and having an m/e of 848. The product was identified as a compound of formula:- $NO_2$ Ph′ CO Ph O Ph O Ph CO Ph O Ph O Ph CO Ph′ $NO_2$, where Ph′ is 1,3-phenylene.

(iii) Bis-4,4′-(3-nitrobenzoyl-4-phenoxy)benzophenone was replaced by the product of step (ii) and the reaction refluxed for 6 hours. The reaction gave a yellow solid (yield 80.6%) melting in the range 231.2-234.2° C and having an m/e of 788.

The product was identified as being the compound of formula VIII in which Z is $-NH_2$.

## Example VI

The diamine prepared according to Example I was condensed with pyromellitic dianhydride to form a polyimide as follows:

To a 3-necked round bottom flask fitted with $N_2$ inlet, stainless steel mechanical stirrer and a drying tube was added 0.02m, 11.45g of the diamine of formula IV, prepared as described in Example 1, together with 80 ml of dimethylacetamide in which it dissolved. To this stirred solution was added pyromellitic dianhydride (0.02m, 4.36g) in a single portion, the stirring being continued for a further three hours at room temperature. This viscous solution was precipitated into water contained in a Waring Blendor to give a suspended powder which was recovered by filtration, and washed with three portions of demineralised water. The powder was then dried in a vacuum oven at 80° C overnight and then heated to 200° C for seven hours under vacuum.

A sample of the polymer was compression moulded at 350° C to give a clear yellow moulding which had an inherent viscosity of 0.36 (0.5% solution in concentrated $H_2SO_4$) and a glass transition temperature of 257° C (DSC onset).

## Example VII

The diamine prepared according was to Example II condensed with pyromellitic dianhydride to form a polyimide as follows:-

The assembly described in Example VI for preparing the polymer was charged with 0.0125m, 7.21g of a diamine of formula V, prepared as described in Example II, together with 30 mls of dimethylacetamide in which it dissolved. With stirring pyromellitic dianhydride (0.0125m, 2.73g) was added in a single portion and washed through with a further 10 ml of dimethylacetamide. Stirring was continued for three hours at room temperature before the polymer was isolated and converted to polyimide in a similar fashion to that described in Example VI.

A compression film moulded at 350° C was creasable, ie tough, and has a glass transition temperature of 258° C as measured by differential scanning calorimetry (DSC).

## Example VIII

The assembly and reagents for making the polymer as described in Example VII were used except that pyromellitic dianhydride was replaced by benzophenone dianhydride (0.0125m and 4.03g). The resulting polymer was compression moulded at 350° C to give a tough film which had a glass transition temperature of 223° C by DSC.

## Example IX

Bis-4,4′-(3-aminobenzoyl-4′-phenoxy)benzophenone (15.72g, 0.026m), diphenylsulphone (50g) and toluene (35ml) were charged into a 250ml flask fitted with an $N_2$ inlet, a mechanical stirrer and a Dean/Stark trap. The contents of the flask were heated to 85° C and pyromellitic anhydride (5.45g, 0.025m) was added and washed in with toluene. The resulting orange slurry was stirred for 30 minutes whilst the temperature was maintained at 85° C. Phthalic anhydride (0.4444g, 0.003m) was then added to the flask. The temperature was raised to and held at 135° C and the reaction mixture refluxed for 2 hours with continuous

removal of the water. When all the water had been removed the temperature of the reaction mixture was slowly raised to and held at 250 °C and refluxed to remove the toluene. Thereafter the reaction mixture was held at 250 °C for a further hour and then raised to and held at 270 °C for 1 hour.

The reaction mixture was then poured onto an aluminium tray and allowed to cool. The resulting diphenylsulphone/polymer mixture was broken up using a hammer mill and the diphenylsulphone removed by washing four times with 500m1 of acetone. The polymer was dried for 8 hours at 80 °C followed by 1 hour at 300 °C in vacuo. The resulting polymer was compression moulded at 350 °C into dark flexible films. The glass transition temperature of the polymer, determined by DSC, was 216 °C.

Comparative Example

Example IX was repeated except the meta substituted material, bis 4,4′-(3-aminobenzoyl-4′-phenoxy)-benzophenone, was replaced by bis 4,4′-(4-aminobenzoyl-4′-phenoxy)benzophenone, the para substituted material.

Attempts to compression mould the resulting polymer at 420 °C and 20 tonnes pressure were unsuccessful. DSC analysis gave a Tm (temperature at which the main peak of melting endotherm is observed) of 483 °C.

These results clearly illustrate that the polymer prepared from the meta diamine according to the invention is easier to process than the polymer prepared from the para diamine.

The DSC equipment used in the examples was a Mettler DSC 20.

**Claims**

1. An aromatic compound of formula I
Z Ph′ X Ph Y Ar Y Ph X Ph′ Z I
wherein
X is selected from -CO-, -SO- or $-SO_2-$;
Y is selected from -O- or -S-;
Z is $-NH_2$ or a maleimide radical;
Ar is a phenylene unit or a polyaromatic group containing at least two aromatic units which are either linked directly together or are linked by a class selected from -CO-, -SO-, $-SO_2-$, -O-, -S-, $-C(CH_3)_2$ and other aliphatic groups;
Ph is 1,4-phenylene; and
Ph′ is 1,3-phenylene when Z is $-NH_2$ and Ph′ is 1,3 - or 1,4 - phenylene when Z is a maleimide radical.

2. An aromatic compound according to claim 1 wherein Ar is selected from -Ph-, -Ph Ph-, -Ph CO Ph-, -Ph $SO_2$ Ph- and -Ph O Ph CO Ph O Ph-.

3. An aromatic compound according to claim 1 selected from

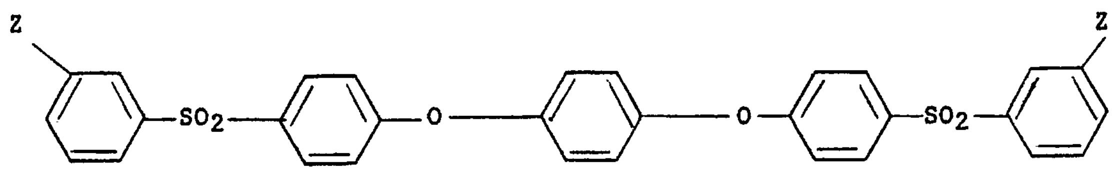

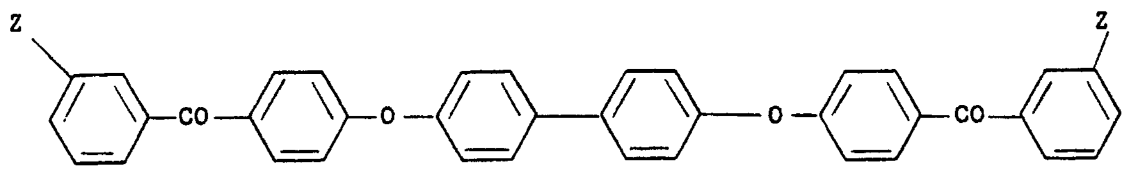

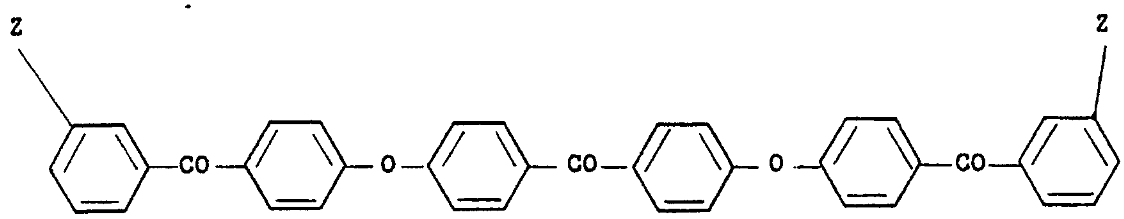

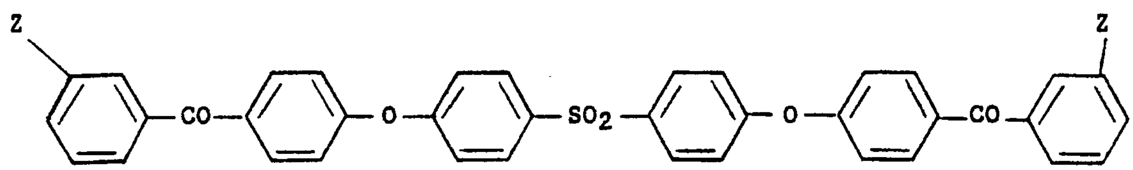

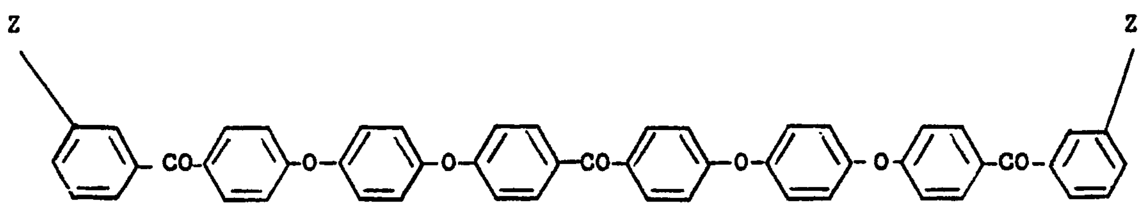

where Z is $-NH-_2$.

4. A process for preparing an aromatic compound as defined in claim 1 where Z is $-NH_2$ which comprises condensing monomer pairs selected from the following groups:-

(i) a halobenzene of formula $H_2$ N Ph′ X Ph Z′ and a bisphenol of formula HO Ar OH;

(ii) a halobenzene of formula $O_2$ N Ph′ X Ph Z′ and a bisphenol of formula HO Ar OH, followed by reduction of the nitro groups to amine groups; and

(iii)an amino-hydroxy compound of formula $H_2$ N Ph′ X Ph OH and a dihalide of formula Z′ Ar Z′, where Ar includes the group $-SO_2$-or -CO- or the condensation is carried out in the presence of a copper catalyst; where Z′ is a halogen and can be the same or different.

5. A process for preparing an aromatic compound as defined in claim 1 where Z is $-NH_2$ which comprises:-

(i) acylating a compound of formula Ph Y Ar Y Ph with a compound of formula $O_2$ N Ph′ X Z′, where Z′ is a halogen, in the presence of an acid catalyst to form a compound of formula

$O_2N\ Ph'\ X\ Ph\ Y\ Ar\ Y\ Ph\ X\ Ph'NO_2$ ;and

(ii) reducing the nitro groups to amine groups.

6. A process for preparing an aromatic compound as defined in claim 1 where Z is a maleimide radical which comprises condensing an aromatic compound as defined in claim 1, where Z is $-NH_2$, with maleic anhydride or a reactive derivative thereof.

7. A thermoplastic aromatic polymer comprising a repeat unit of formula

$- D - Z^3 -$

wherein D is a divalent aromatic radical derived from a compound of formula I, as defined in claim 1 and in which Z is $-NH_2$ and Ph′ is 1,3-phenylene, and $Z^3$ is

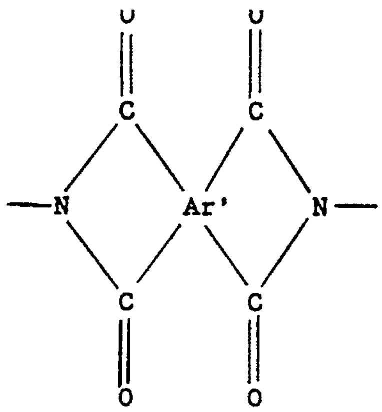

or

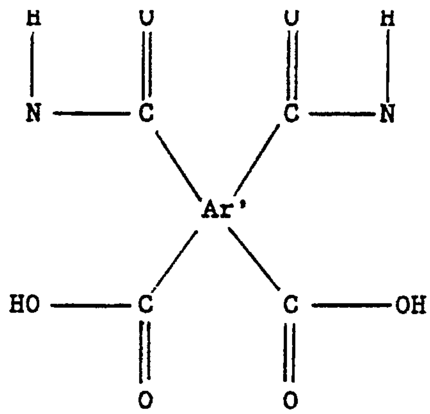

in which Ar′ is a tetravalent aromatic radical derived from a dianhydride or a corresponding derivative thereof, and wherein up to 50 mole % of the radicals D are optionally replaced by divalent organic radicals derived from diamines other than those falling within formula I.

8. A process for preparing a polymer as defined in claim 7 which comprises reacting a dianhydride of formula

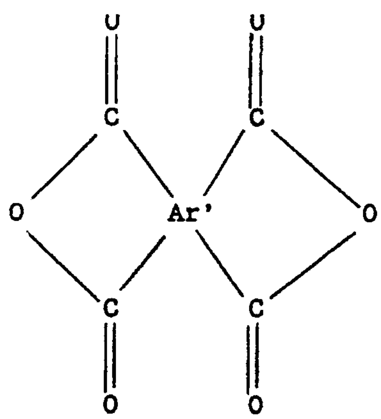

or a corresponding derivative thereof, where Ar′ is a tetravalent aromatic radical derived from a dianhydride or a corresponding derivative thereof, with at least one compound of formula I as defined in claim 1 and in which Z is $-NH_2$ and Ph′ is 1,3-phenylene.

9. An epoxy resin system comprising an aromatic compound as defined in claim 1, where Z is $-NH_2$, as a cross-linking agent.

10. A shaped article comprising an aromatic compound as claimed in claim 1, where Z is a maleimide radical, when thermoset.

11. A shaped article comprising a polymer as claimed in claim 7.